# EUROPEAN PATENT APPLICATION

(11) **EP 3 858 381 A1**
(43) Date of publication of application: **04.08.2021**
(21) Application number: 21162470.5
(22) Date of filing: 03.02.2012
(51) Int. Cl.: A61K 39/295, C07K 14/235, A61K 39/10, A61K 39/02, A61K 39/12, A61P 11/00, A61P 31/00, A61P 31/04, A61P 31/14, A61P 31/16, A61P 31/12

(54) **IMMUNOGENIC BORDETELLA BRONCHISEPTICA COMPOSITIONS**

(30) Priority: 04.02.2011 US 201161439597 P
(62) Divisional of application: 12705438.5
(71) Applicant: Zoetis Services LLC, Parsippany, NJ 07054 (US)
(72) Inventor: ABDELMAGID, Omar, Yousif, Kalamazoo, MI 49007 (US); Bricker, Joseph Michael, Kalamazoo, MI 49007 (US); Shields, Shelly Lynn, Kalamazoo, MI 49007 (US); Galvin, Jeffrey E., Lincoln, MI 68521 (US)
(74) Representative: Mannion, Sally Kim

(57) **Abstract**

Provided herein are compositions, combinations, and methods comprising *Bordetella bronchiseptica* and isolated pertactin, which are effective in treating or preventing respiratory infections, such as kennel cough, in animals.

## Description

### FIELD OF THE INVENTION

The present invention relates to the field of immunology, and in particular to the field of immunogenic and vaccine compositions. More specifically, the present disclosure relates to compositions comprising a *Bordetella bronchiseptica* preparation in combination with pertactin for treatment or prevention of respiratory disease(s) in a dog.

### BACKGROUND OF THE INVENTION

*Bordetella bronchiseptica* is a gram-negative bacteria that colonizes in the respiratory tract of dogs and causes tracheobonchitis or "kennel cough". Hawkins, E.C., Veterinary Internal Medicine (1995), pp. 767-811. *Bordetella bronchiseptica* predisposes dogs to the influence of other respiratory agents, and frequently exists concurrently with them. To date, a number of vaccines are available for treatment of tracheobonchitis caused by *Bordetella bronchiseptica,* including Nobivac®, Bronchi-Shield®, Bronchicine® CAe, Vanguard® B, Univac 2, Recombitek® KC2, Naramune™-2 and Kennel-Jec™ 2.

However, the majority of existing commercial vaccines require cumbersome intranasal administration as well as the addition of adjuvants, which can result in deleterious side-effects, such as burning and irritation. Viera Scheibner et al., Nexus Dec 2000 (Vol 8, No1). Intranasal vaccines are unpopular with veterinary practitioners due to their personal risk with fractious animals during vaccine administration. Subunit vaccines, such as those involving the use of p68 protein of *Bordetella bronchiseptica* (pertactin), have been explored but to date have not been included in any commercial canine vaccines, possibly due to insufficient immunogenicity, reactivity and/or formulation stability.

*Bordetella bronchiseptica* is a significant factor in canine infectious respiratory disease complex (CIRDC), which is a highly contagious disease common in dogs housed in crowded conditions, such as re-homing centers and boarding or training kennels. The pathogenesis of CIRDC is considered to be multifactorial, involving several viruses and bacteria. Infectious agents known to be causative agents of CIRDC include, in addition to the bacterium *Bordetella bronchiseptica* (Bemis et al., Lab. Anim. Sci., 29:48-52, 1977), canine respiratory coronavirus (CRCoV) (Erles et al., Virology, 310(2):216-223, 2003), canine influenza virus (CIV) (Crawford et al., Science, 310(5747):482-485, 2005), canine parainfluenzavirus (CPIV) (Binn et al., Exp. Biol. Med., 126:140-145, 1967), Mycoplasma cynos (Chalker et al., Microbiology, 150:3491-3497, 2004) and canine adenovirus serotype 2 (CAV-2) (Ditchfield et al., Can. Vet. J., 3:238-247, 1962). To date, no comprehensive combination vaccine against all, or the majority, of the aforementioned pathogens has emerged.

Accordingly, there remains a need for an immunogenic composition capable of being safely administered parenterally to a dog, which provides long-acting immuno-protection against *Bordetella bronchiseptica* without deleterious side-effects or interference with other antigens in a combination vaccine or causing risk to the veterinary practitioner. The present disclosure fulfills these and other related needs.

### SUMMARY OF THE INVENTION

In one embodiment the present invention provides an immunogenic composition comprising, *inter alia, Bordetella bronchiseptica* and an isolated pertactin antigen. In another embodiment, said pertactin antigen is a recombinant protein. In another embodiment, said pertactin antigen is from *Bordetella bronchiseptica.* In another embodiment, said pertactin antigen is p68. In another embodiment, said composition further comprises an isolated Bsp22 antigen.

In another embodiment, the *Bordetella bronchiseptica* component is a bacterin or a bacterial extract. In another embodiment, said composition is non-adjuvanted. In another embodiment, said composition further comprises an adjuvant.

In another embodiment, said pertactin antigen is present at between about 1 µg and about 30 µg. More particularly, said pertactin is present at between about 5 µg and about 20 µg, more particular still, at between about 7 µg and about 15 µg, and even more particularly, at about 5 µg, 10 µg, 15 µg or 20 µg. Preferably, said pertactin antigen is prepared by solubilizing pertactin inclusion bodies in urea and optionally purifying by column chromatography. Said pertactin antigens are soluble and preferably substantially free of aggregates.

Another embodiment provides an immunogenic composition comprising a plurality of recombinant p68 pertactin antigens, wherein said composition is substantially free of p68 aggregates. More particularly, said plurality of recombinant p68 pertactin antigens are prepared by solubilizing pertactin inclusion bodies in urea and optionally purifying by column chromatography. The invention also provides methods of making p68 pertactin antigens by solubilizing pertactin inclusion bodies in urea and optionally purifying by column chromatography.

In another embodiment, said composition is formulated for parenteral administration such that it further comprises a diluent or excipient for parenteral administration to a dog.

In another embodiment, said composition further comprises an antigen from a canine respiratory pathogen selected from the group consisting of: canine parainfluenza virus (CPIV), canine adenovirus-2 (CAV-2), canine respiratory coronavirus (CRCoV) and canine influenza virus (CIV). In a more particular embodiment, said composition comprises at least two, three or four of the antigens from the canine respiratory pathogens.

In another embodiment, the immunogenic compositions described herein do not comprise non-respiratory antigens. Thus, one embodiment of the invention provides a composition as described herein with the proviso that it does not include a non-respiratory antigen. The non-respiratory antigens do not cause respiratory disease in a subject. Non-limiting examples of such non-respiratory antigens include rabies virus, canine parvovirus, enteric canine coronavirus, *Leptospira* species, and *Borrelia burgdorferi.*

In another embodiment, said antigen is from canine respiratory coronavirus (CRCoV). In another embodiment, said antigen is from canine influenza virus (CIV). In another embodiment, said antigen is from canine parainfluenza virus (CPIV). In another embodiment, said antigen is from canine adenovirus-2 (CAV-2). In another embodiment, said antigen is from canine parainfluenza virus (CPIV) and canine adenovirus-2 (CAV-2).

In another embodiment, said composition comprises antigens from canine parainfluenza virus (CPIV), canine adenovirus-2 (CAV-2), canine respiratory coronavirus (CRCoV) and canine influenza virus (CIV).

In another embodiment, the composition induces an immune response to *Bordetella bronchiseptica* and at least one of canine parainfluenza virus (CPIV), canine adenovirus-2 (CAV-2), canine respiratory coronavirus (CRCoV) and canine influenza virus (CIV).

Another embodiment of the invention provides an immunogenic composition comprising canine respiratory corona virus (CRCoV), a *Bordetella bronchiseptica* preparation and canine influenza virus (CIV). More particularly, said composition comprises an isolated pertactin antigen.

Another embodiment of the present invention provides a method or use of the immunogenic composition of any one of the foregoing embodiments for treatment or prevention of infection from a canine respiratory pathogen in a dog. In another embodiment, said composition prevents infection from said canine respiratory pathogen in said dog for a period of 6-months or more. In another embodiment, said composition prevents infection from said canine respiratory pathogen in said dog for a period of about 1-year.

In another embodiment, the canine respiratory pathogen is *Bordetella bronchiseptica.* In another embodiment, the canine respiratory pathogen further comprises at least one, two, three or four of: canine parainfluenza virus (CPIV), canine adenovirus-2 (CAV-2), canine respiratory coronavirus (CRCoV) and canine influenza virus (CIV).

Another embodiment of the present invention provides a method or use of the immunogenic composition of any one of the foregoing embodiments for treatment or prevention of canine infectious respiratory disease complex (CIRDC), wherein the composition treats or prevents infection from a plurality of canine respiratory pathogens. Another embodiment provides for the use or method of parenterally administering an immunongenic composition as described herein.

Another embodiment provides for the manufacture of a medicament comprising the immunogenic composition for treatment or prevention of infection from a canine respiratory pathogen in a dog.

These and other embodiments, features, and advantages of the invention will become apparent from the detailed description and the appended claims set forth herein below. It is understood that each of the foregoing and following embodiments can be combined into a single embodiment.

### DETAILED DESCRIPTION OF THE INVENTION

The definitions below apply to this disclosure. They supersede any contradictory definitions contained in each individual reference incorporated herein by reference. Words not defined have the meaning commonly used by one skilled in the art. Further, unless otherwise required by context, singular terms shall include pluralities and plural terms shall include the singular.

"About" or "approximately," when used in connection with a measurable numerical variable, refers to the indicated value of the variable and to all values of the variable that are within the experimental error of the indicated value (e.g., within the 95% confidence interval for the mean), or within 10 percent of the indicated value, whichever is greater. If "about" is used in reference to time intervals in weeks, "about 3 weeks" is 17 to 25 days, and "about 2 to about 4 weeks" is 10 to 40 days.

"Adjuvant", as used herein, refers to any substance which serves as a non-specific stimulator of the immune response. See below for a further description of adjuvants.

The term "animal", as used herein, includes any animal that is susceptible to infection from *Bordetella bronchiseptica* and/or canine respiratory disease complex, including mammals, both domesticated and wild. Preferably, animal as used herein refers to a dog or a human.

"Antibody", as used herein, is any polypeptide comprising an antigen-binding site regardless of the source, method of production, or other characteristics. It refers to an immunoglobulin molecule or a fragment thereof that specifically binds to an antigen as the result of an immune response to that antigen. Immunoglobulins are serum proteins composed of "light" and "heavy" polypeptide chains having "constant" and "variable" regions and are divided into classes (e.g., IgA, IgD, IgE, IgG, and IgM) based on the composition of the constant regions. An antibody that is "specific" for a given antigen indicates that the variable regions of the antibody recognize and bind a specific antigen exclusively. The term includes, but is not limited to: a polyclonal antibody, a monoclonal antibody, a monospecific antibody, polyspecific antibody, humanized antibody, a tetrameric antibody, a tetravalent antibody, a multispecific antibody, a single chain antibody, a domain-specific antibody, a single domain antibody, a domain-deleted antibody, a fusion protein, an ScFc fusion protein, a single-chain antibody, chimeric antibody, synthetic antibody, recombinant antibody, hybrid antibody, mutated antibody, and CDR-grafted antibodies. Antibodies can be intact immunoglobulins derived from natural sources or from recombinant sources, or can be immunoreactive portions of intact immunoglobulins. An "antibody" can be converted to an antigen-binding protein, which includes but is not limited to antibody fragments which include but are not limited to: Fab, F(ab')₂, an Fab' fragment, an Fv fragment, a single-chain Fv (ScFv) fragment, an Fd fragment, a dAb fragment, diabodies, a CDR3 peptide, a constrained FR3-CDR3-FR4 peptide, a nanobody, a bivalent nanobody, a small modular immunopharmaceutical (SMIPs), and a minibody and any of above mentioned fragments and their chemically or genetically manipulated counterparts, as well as other antibody fragments that retain antigen-binding function. Typically, such fragments would comprise an antigen-binding domain. As will be recognized by those of skill in the art, any of such molecules may be engineered (for example "germlined") to decrease its immunogenicity, increase its affinity, alter its specificity, or for other purposes.

"Antigen" or "immunogen", as used herein, refers to a molecule that contains one or more epitopes (linear, conformational or both) that upon exposure to a subject will induce an immune response that is specific for that antigen. An epitope is the specific site of the antigen which binds to a T-cell receptor or specific antibody, and typically comprises about 3 amino acid residues to about 20 amino acid residues. The term antigen refers to subunit antigens-antigens separate and discrete from a whole organism with which the antigen is associated in nature-as well as killed, attenuated or inactivated bacteria, viruses, fungi, parasites or other microbes. The term antigen also refers to antibodies, such as anti-idiotype antibodies or fragments thereof, and to synthetic peptide mimotopes that can mimic an antigen or antigenic determinant (epitope). The term antigen also refers to an oligonucleotide or polynucleotide that expresses an antigen or antigenic determinant in vivo, such as in DNA immunization applications.

"Antigenicity", as used herein, refers to the capability of a protein or polypeptide to be immunospecifically bound by an antibody raised against the protein or polypeptide.

The term *"Bordetella bronchiseptica"* or *"B. bronchiseptica"* refers to: a live attenuated bacterium of *Bordetella bronchiseptica,* a killed whole cell extract (bacterin) of *Bordetella bronchiseptica* or a cellular bacterial extract of *Bordetella bronchiseptica.*

"Buffer" means a chemical system that prevents change in the concentration of another chemical substance. Proton donor and acceptor systems serve as buffers, preventing marked changes in hydrogen ion concentration (pH). A further example of a buffer is a solution containing a mixture of a weak acid and its salt (conjugate base), or a weak base and its salt (conjugate acid).

"Canine", as used herein, includes what is commonly called the dog, but includes other members of the family *Canidae.*

The term "cell line" or "host cell", as used herein, means a prokaryotic or eukaryotic cell in which a virus can replicate or be maintained.

The term "culture", as used herein, means a population of cells or microorganisms growing in the absence of other species or types.

"Dose" refers to a vaccine or immunogenic composition given to a subject. A "first dose" or "priming dose" refers to the dose of such a composition given on Day 0. A "second dose" or a "third dose" or an "annual dose" refers to an amount of such composition given subsequent to the first dose, which can be but is not required to be the same vaccine or immunogenic composition as the first dose.

An "epitope" is the specific site of the antigen which binds to a T-cell receptor or specific antibody, and typically comprises from about 3 amino acid residues to about 20 amino acid residues.

"Excipient", as used herein, refers to a non-reactive carrier component of a vaccine or immunogenic composition that is not an antigen. Preferred excipients are those known in the art for parenteral injection.

"Fragment" refers to a truncated portion of a protein or gene. "Functional fragment" and "biologically active fragment" refer to a fragment that retains the biological properties of the full length protein or gene.

"Homology" or "percent homology" refers to the percentage of nucleotide or amino acid residues in the candidate sequence that are identical or similar with the residues in the comparator sequence(s) after aligning the sequences and introducing gaps, if necessary, to achieve the maximum percent sequence homology, and also considering any conservative substitutions as part of the sequence homology.

"Homologs" or "species homologs" include genes found in two or more different species which possess substantial polynucleotide sequence homology, and possess the same, or similar, biological functions and/or properties. Preferably polynucleotide sequences which represent species homologs will hybridize under moderately stringent conditions, as described herein by example, and possess the same or similar biological activities and/or properties. In another aspect, polynucleotides representing species homologs will share greater than about 60% sequence homology, greater than about 70% sequence homology, greater than about 80% sequence homology, greater than about 90% sequence homology, greater than about 95% sequence homology, greater than about 96% sequence homology, greater than about 97% sequence homology, greater than about 98% sequence homology, or greater than about 99% sequence homology.

"Identity" or "percent identity" refers to the percentage of nucleotides or amino acids in the candidate sequence that are identical with the residues in the comparator sequence after aligning both sequences and introducing gaps, if necessary, to achieve the maximum percent sequence identity, and not considering any conservative substitutions as part of the sequence identity.

"Immune response", as used herein, in a subject refers to the development of a humoral immune response, a cellular immune response, or a humoral and a cellular immune response to an antigen. A "humoral immune response" refers to one that is at least in part mediated by antibodies. A "cellular immune response" is one mediated by T-lymphocytes or other white blood cells or both, and includes the production of cytokines, chemokines and similar molecules produced by activated T-cells, white blood cells, or both. Immune responses can be determined using standard immunoassays and neutralization assays, which are known in the art.

"Immunogenicity", as used herein, refers to the capability of a protein or polypeptide to elicit an immune response directed specifically against a bacteria or virus that causes the identified disease.

An "immunogenic composition" is a preparation containing an immunogen, including, e.g., a protein, a peptide, a whole cell, inactivated, subunit or attenuated virus, or a polysaccharide, or combination thereof, administered to stimulate the recipient's humoral and cellular immune systems to one or more of the antigens present in the immunogenic composition. "Immunization" is the process of administering an immunogenic composition and stimulating an immune or immunogenic response to an antigen in a host. Preferred hosts are mammals, such as dogs. Preferably, the immunogenic composition is a vaccine.

"Immunologically protective amount", as used herein, is an amount of an antigen effective to induce an immunogenic response in the recipient that is adequate to prevent or ameliorate signs or symptoms of disease, including adverse health effects or complications thereof. Either humoral immunity or cell-mediated immunity or both can be induced. The immunogenic response of an animal to a composition can be evaluated, e.g., indirectly through measurement of antibody titers, lymphocyte proliferation assays, or directly through monitoring signs and symptoms after challenge with wild type strain. The protective immunity conferred by a composition or vaccine can be evaluated by measuring, e.g., reduction of shed of challenge organisms, reduction in clinical signs such as mortality, morbidity, temperature, and overall physical condition, health and performance of the subject. The immune response can comprise, without limitation, induction of cellular and/or humoral immunity. The amount of a composition or vaccine that is therapeutically effective can vary, depending on the particular organism used, or the condition of the animal being treated or vaccinated, and can be determined by a veterinarian.

"Intranasal" administration, as used herein, refers to the introduction of a substance, such as a vaccine or other composition, into a subject's body through or by way of the nose, and involves transport of the substance primarily through the nasal mucosa.

The term "isolated" refers to a substance that is either in substantially pure form, for example, greater than about 95% purity; or purified or enriched in some way from its natural environment. Reference to "isolated pertactin" indicates a pertactin protein that is removed from its natural environment, such as from a host animal/dog, in a growth media, or purified from a whole cell *Bordetella bronchiseptica* preparation and may subsequently be added back to a composition comprising *Bordetella bronchiseptica* extract (i.e. spiked with pertactin isolates). The term "isolated" encompasses immunogens that are in solution with other agents/diluents/excipients/adjuvants/proteins.

"Medicinal agent" refers to any agent which is useful in the prevention, cure, or improvement of a medical condition, or the prevention of some physiological condition or occurrence.

"Monoclonal antibody", as used herein, refers to antibodies produced by a single line of hybridoma cells, all directed towards one epitope on a particular antigen. The antigen used to make the monoclonal antibody can be provided as an isolated protein of the pathogen or the whole pathogen. A "hybridoma" is a clonal cell line that consists of hybrid cells formed by the fusion of a myeloma cell and a specific antibody-producing cell. In general, monoclonal antibodies are of mouse origin. However, monoclonal antibody also refers to a clonal population of an antibody made against a particular epitope of an antigen produced by phage display technology, or method that is equivalent to phage display, or hybrid cells of non-mouse origin.

"Oral" or "peroral" administration, as used herein, refers to the introduction of a substance, such as a vaccine or other composition, into a subject's body through or by way of the mouth and involves swallowing or transport through the oral mucosa (e.g., sublingual or buccal absorption) or both. Intratracheal is also a means of oral or peroral administration.

"Oronasal" administration, as used herein, refers to the introduction of a substance, such as a composition or vaccine, into a subject's body through or by way of the nose and the mouth, as would occur, for example, by placing one or more droplets in the nose. Oronasal administration involves transport processes associated with oral and intranasal administration.

"Parenteral administration", as used herein, refers to the introduction of a substance, such as a composition or vaccine, into a subject's body through or by way of a route that does not include the digestive tract. Parenteral administration includes subcutaneous, intramuscular, intraarterial, and intravenous administration. For the purposes of this disclosure, parenteral administration excludes administration routes that primarily involve transport of the substance through mucosal tissue in the mouth, nose, trachea, and lungs.

The term "pathogen" or "pathogenic microorganism", as used herein, means a microorganism - for example CPIV, CAV-2, CRCoV, Mycoplasma cynos, CIV, or *Bordetella bronchiseptica* - which is capable of inducing or causing a disease, illness, or abnormal state in its host animal, preferably a respiratory disease, such as CIRDC.

"Pertactin", as used herein, refers to an outer membrane protein of *Bordetella.* Preferably, the pertactin is from *B. bronchiseptica* and most preferably, "p68", and is encoded by the gene, *prnA.* Pertactin can be isolated in its native form from *Bordetella bronchiseptica,* or it can be produced recombinantly. Sequences and examples of pertactin are provided in U.S. Patent No. 7,736,658, the contents of which is hereby incorporated by reference. The pertactin antigen used herein includes lipidated forms of the protein.

"Pharmaceutically acceptable" refers to substances which, within the scope of sound medical judgment, are suitable for use in contact with the tissues of subjects without undue toxicity, irritation, allergic response, and the like, commensurate with a reasonable benefit-to-risk ratio, and effective for their intended use.

"Polyclonal antibody", as used herein, refers to a mixed population of antibodies made against a particular pathogen or antigen. In general, the population contains a variety of antibody groups, each group directed towards a particular epitope of the pathogen or antigen. To make polyclonal antibodies, the whole pathogen, or an isolated antigen, is introduced by inoculation or infection into a host, which induces the host to make antibodies against the pathogen or antigen.

The term "polynucleotide", as used herein, means an organic polymer molecule composed of nucleotide monomers covalently bonded in a chain. DNA (deoxyribonucleic acid) and RNA (ribonucleic acid) are examples of polynucleotides with distinct biological function.

The term "polypeptide", as used herein, means an organic polymer molecule composed of two or more amino acids bonded in a chain.

"Preventing infection", as used herein, means to prevent or inhibit the replication of the bacteria or virus which cause the identified disease, to inhibit transmission of the bacteria or virus, to prevent the bacteria or virus from establishing itself in its host, or to alleviate the symptoms of the disease caused by infection. The treatment is considered therapeutic if there is a reduction in bacterial or viral load.

"Protection", "protecting", "protective immunity", and the like, as used herein with respect to a vaccine or other composition, means that the vaccine or composition prevents or reduces the symptoms of the disease caused by the organism from which the antigen(s) used in the vaccine or composition is derived. The terms "protection", "protecting", and the like, also mean that the vaccine or composition can be used to "treat" the disease, or one or more symptoms of the disease that already exists in a subject.

"Respiratory" administration, as used herein, refers to the introduction of a substance, such as a vaccine or other composition, into a subject's body through or by way of inhalation of a nebulized (atomized) substance. In respiratory administration, the primary transport mechanism involves absorption of the atomized substance through the mucosa in the trachea, bronchi, and lungs and is therefore different than intranasal or peroral administration.

The terms "specific binding," "specifically binds," and the like, are defined as two or more molecules that form a complex that is measurable under physiologic or assay conditions and is selective. An antibody or other inhibitor is said to "specifically bind" to a protein if, under appropriately selected conditions, such binding is not substantially inhibited, while at the same time non-specific binding is inhibited. Specific binding is characterized by high affinity and is selective for the compound or protein. Nonspecific binding usually has low affinity. Binding in IgG antibodies, for example, is generally characterized by an affinity of at least about 10⁻⁷ M or higher, such as at least about 10⁻⁸ M or higher, or at least about 10⁻⁹ M or higher, or at least about 10⁻¹⁰ or higher, or at least about 10⁻¹¹ M or higher, or at least about 10⁻¹² M or higher. The term is also applicable where, e.g., an antigen-binding domain is specific for a particular epitope that is not carried by numerous antigens, in which case the antibody carrying the antigen-binding domain will generally not bind other antigens.

"Specific immunogenic fragment", as used herein, refers to a portion of a sequence that is recognizable by an antibody or T cell specific for that sequence.

"Subject", as used herein, refers to any animal having an immune system, which includes mammals, such as dogs.

"Substantially identical", as used herein, refers to a degree of sequence identity of at least about 90%, at least about 95%, at least about 96%, at least about 97%, at least about 98%, or at least about 99%.

"Subunit vaccine", and "subunit composition", as used herein, refers to a type of vaccine or composition that includes an antigens- but not all antigens- which are derived from or homologous to, antigens from a pathogen of interest, such as a virus, bacterium, parasite or fungus. Such a composition or vaccine is substantially free of intact pathogen cells or pathogenic particles, or the lysate of such cells or particles. Thus, a subunit vaccine or subunit composition can be prepared from at least partially purified, or substantially purified, immunogenic polypeptides from the pathogen or their analogs. Methods of obtaining an antigen or antigens in the subunit vaccine or subunit composition include standard purification techniques, recombinant production, or chemical synthesis. A "subunit vaccine" or "subunit composition" thus refers to a vaccine or composition consisting of a defined antigenic component or components of a virus, bacterium, or other immunogen. Preferably, the subunit component of the present invention is recombinantly produced and most preferably it is pertactin (p68).

"TCID₅₀" refers to "tissue culture infective dose" and is defined as that dilution of a virus required to infect 50% of a given batch of inoculated cell cultures. Various methods can be used to calculate TCID₅₀, including the Spearman-Karber method, which is utilized throughout this specification. For a description of the Spearman-Karber method, see B. W. Mahy & H. O. Kangro, Virology Methods Manual 25-46 (1996).

"Therapeutic agent", as used herein, refers to any molecule, compound, virus or treatment, preferably a virus attenuated or killed, or subunit or compound, that assists in the treatment of a viral, bacterial, parasitic or fungal infection, disease or condition caused thereby.

"Therapeutically effective amount", as used herein, refers to an amount of an antigen or vaccine or composition that would induce an immune response in a subject (e.g., dog) receiving the antigen or vaccine or composition which is adequate to prevent or ameliorate signs or symptoms of disease, including adverse health effects or complications thereof, caused by infection with a pathogen, such as a virus, bacterium, parasite or fungus. Humoral immunity or cell-mediated immunity, or both humoral and cell-mediated immunity, can be induced. The immunogenic response of an animal to an antigen, vaccine, or composition can be evaluated indirectly through measurement of antibody titers, lymphocyte proliferation assays, or directly through monitoring signs and symptoms after challenge with the wild type strain. The protective immunity conferred by a vaccine or composition can be evaluated by measuring reduction of challenge organism shed, and/or reduction in clinical signs, such as mortality, morbidity, temperature, and overall physical condition, health, and performance of the subject. The amount of a vaccine or composition that is therapeutically effective can vary, depending on the particular immunogen used, or the condition of the subject, and can be determined by one skilled in the art.

"Treat" or "treating", as used herein, refers to reversing, alleviating, inhibiting the progress of, or preventing a disorder, condition or disease to which such term applies, or to preventing one or more symptoms of such disorder, condition or disease.

"Treatment", as used herein, refers to the act of "treating", as defined immediately above.

"Vaccine" or "vaccine composition," as used herein, refers to an immunogenic composition selected from a virus or bacteria, either modified live, attenuated, or killed, or a subunit vaccine, or any combination of the aforementioned. Administration of the vaccine to a subject results in an immune response. The vaccine can be introduced directly into the subject by any known route of administration, including parenterally, perorally, and the like. The terms mean a composition which prevents or reduces an infection, or which prevents or reduces one or more signs or symptoms of infection. The protective effects of a vaccine composition against a pathogen are normally achieved by inducing in the subject an immune response. Generally speaking, abolished or reduced incidences of infection, amelioration of the signs or symptoms, or accelerated elimination of the microorganism from the infected subjects are indicative of the protective effects of a vaccine composition. The vaccine compositions of the present invention provide protective effects against infections caused by canine respiratory disease pathogens.

"Veterinarily acceptable", as used herein, refers to substances which are, within the scope of sound medical judgment, suitable for use in contact with the tissues of veterinary subjects without undue toxicity, irritation, allergic response, and the like, commensurate with a reasonable benefit-to-risk ratio, and effective for their intended use.

"Veterinarily acceptable carrier", as used herein, refers to a carrier medium that does not interfere with the effectiveness of the biological activity of the active ingredient, and is not toxic to the veterinary subject to whom it is administered.

### Antigens, Immunogenic Compositions, and Vaccines

The present disclosure is based upon the unexpected discovery that inclusion of isolated pertactin antigens in conjunction with a *Bordetella bronchiseptica* preparation results in substantially improved efficacy and safety. That is, when administered parenterally to a dog prior to bacterial challenge, the composition prevents onset of tracheobonchitis and does not result in adverse side-effects.

The present invention also provides immunogenic compositions and vaccines comprising one or more viruses and bacteria or subunits that are suitable for administration to a canine for treatment against CIRDC. The canine respiratory coronavirus (CRCoV) encompassed by this invention can be characterised as a coronavirus present in the respiratory tracts of dogs with infectious respiratory disease. CRCoV is phylogenetically most closely related to bovine coronavirus (BCoV), human coronavirus (HCoV) strain OC43 and hemagglutinating encephalomyelitis virus (HEV); enteric canine coronavirus (CCoV) is only distantly related to CRCoV. A representative example of a CRCoV suitable for use in the present invention includes a strain identified as CRCoV strain 4182 (Erles et al., Virus Res., 124:78-87, 2007).

The influenza virus antigens encompassed by this invention can be any identified influenza virus strain, from any bird or mammal, including but not limited to, influenza virus having the subtype H3 hemagglutinin and subtype N8 neuraminidase, or the H3N8 subtype, more commonly referred to as an H3N8 virus. The influenza can be of mammalian or avian origin, including but not limited to swine, equine or canine origin. In one embodiment a canine influenza antigen is used. In one embodiment an equine influenza antigen is used. In one embodiment, a strain having the subtype glycoproteins designated H3 or N8 is used. In one embodiment, a strain having both subtype H3 and N8 glycoproteins is used.

The influenza antigens encompassed by this invention can be isolated from dogs, horses, pigs, and fowl, both domestic and wild. The animals chosen for sample collection should display acute and/or sub-acute clinical syndromes, which can include mild to severe respiratory symptoms and fever. Animals can also exhibit signs of anorexia and lethargy. Methods of virus isolation are well known to those skilled in the art including: inoculating mammalian or avian cell cultures, inoculating embryonated eggs with nasal or pharyngeal mucus samples from clinical specimens, collection by swabbing of the nasal passage or throat, or by collecting tissues such as spleen, lung, tonsil and liver and lung lavage. The cytopathic effect of the virus can be observed in cell culture. Allantoic fluid or cell lysates can be tested for their ability to agglutinate human, chicken, turkey or guinea pig red blood cells, presumptive evidence for the presence of an influenza virus.

A representative example of a canine influenza virus (CIV) strain suitable for use in the present invention includes a strain identified as A/canine/lowa/9A1/B5/08/D12, which was deposited as PTA-7694 on 29 June 2006 at the American Type Culture Collection (ATCC), 10801 University Boulevard, Manassas, VA 20110-2209. A representative strain of the CIV antigen is the CIV virus strain in the commercial vaccine, Vanguard@ CIV (Pfizer). This invention also encompasses vaccines comprising a strain identified as Equine Influenza Strain A/Equine/2/Miami/1/63. This strain is deposited at the ATCC, with accession number VR 317. Additional examples of influenza viruses for use in the present invention are A/canine/lowa/13628/2005, A/Equine/Kentucky/1998, A/Equine/Kentucky/15/2002, A/Equine/Ohio/1/2003, A/Equine/Kentucky/1/1994, A/Equine/Massachusetts/213/2003, A/Equine/Wisconsin/2003, A/Equine/NewYork/1999, and A/Equine/Newmarket/A2/1993. Other preferred strains and/or isolates of CIV include those disclosed in U.S. Patent Nos. 7,959,929 (particularly strains and HA sequences identified therein as Jacksonville/2005, Miami/2005, FL/242/03 and Florida/43/04), 7,384,642, 7,572,620 and 7,468,187, the contents of which, including all sequences, particularly HA sequences, and strains, are hereby incorporated by reference as if set forth fully herein. Additonally, a CIV strain suitable for use herein includes the Colorado CIV isolate described in Barrell et al., J. Vet. Intern. Med., 24 (6), 1524-1527 (2010), having accession number ADW41784.

The canine parainfluenza virus (CPIV) encompassed by this invention can be characterized as one of the viruses known to be a causative agent associated with kennel cough. A representative strain of the CPIV antigen is the attenuated CPI virus strain in the commercial vaccine, Vanguard® Plus 5 (Pfizer). Another representative strain of the CPIV antigen is the attenuated CPI virus strain having the designation of "NL-CPI-5" (National Veterinary Service Laboratory, Ames, IA).

The canine adenovirus, type 2 (CAV-2) encompassed by this invention can be characterized as one of the viruses also known to be a causative agent associated with kennel cough. A representative strain of the CAV-2 antigen is the attenuated CAV-2 virus strain in the commercial vaccine, Vanguard® Plus 5 (Pfizer). A representative strain of the CAV-2 antigen is the attenuated CAV-2 strain designated as the "Manhattan" strain (National Veterinary Service Laboratory, Ames, IA).

The Mycoplasma cynos (M. cynos) encompassed by this invention is described in Chalker et al., Microbiology, 150:3491-3497, 2004 and is the only species of mycoplasma commonly associated with respiratory disease. Immunogenic compositions against M. cynos are described in US 2007/0098739, incorporated herein by reference.

The *Bordetella bronchiseptica* component encompassed by this invention can be characterized as the bacterial causative agent associated with kennel cough. The immunogenic compositions and vaccines encompassed by the present invention can be one or more of: a live attenuated *Bordetella bronchiseptica,* a *Bordetella bronchiseptica* bacterin or a bacterial extract. Additionally, the composition preferably also includes an isolated subunit antigen of *Bordetella bronchiseptica.*

In one embodiment the *Bordetella bronchiseptica* is prepared as a whole cell sonicate purified through column chromatography as provided in Patent Application No. FR2571618, filed October 12, 1984. Another representative example of a *Bordetella bronchiseptica* is the bacterial extract Bronchicine™ CAe (Pfizer), which is prepared from antigenic material extracted from *Bordetella bronchiseptica* cells. Another example of *Bordetella bronchiseptica* is the live attenuated *bronchiseptica* strain B-C2 present in Nobivac® and/or the live *bronchiseptica* strain from Intra-Trac®, Bronchi-Shield®, Naramune™, Recombitek®, Univac, and/or Kennel-Jec™.

Additionally, a subunit is preferably also present (i.e. supplemented), in combination with the *Bordetella bronchiseptica* component. A representative example of the subunit is an isolated pertactin antigen, preferably, a *Bordetella bronchiseptica* p68 antigen, particularly the recombinant *Bordetella bronchiseptica* p68 antigen which is recognized by the p68-specific monoclonal antibody Bord 2-7 (described in US 7,736,658, incorporated herein by reference) and in one preferred embodiment, has an amino acid sequence as set forth in US 7,736,658 or having homology thereto.

The recombinant p68 pertactin antigen is preferably prepared in a soluble form, such that native-like structure is preserved or restored during processing. Accordingly, one aspect of the invention provides a recombinant p68 pertactin that is substantially free (less than about 80%, 90%, 95% or even 99%) of aggregates. In another embodiment the recombinant p68 pertactin is solubilised with urea, preferably about 0.1 M, 0.5 M, 1 M, 2 M, 3 M or 6 M solution of urea. Thereafter, the p68 antigen can be purified, such as through column chromatography. One such solubilisation process is described in *Surinder et al.,* J. Bioscience and Bioengineering, v. 99(4), pgs 303-310 (2005).

Pertactin antigens used herein also include lipidated forms. Examples of production of lipidated proteins is provided in Erdile et al., Infection and Immunity, (1993) v.61(1) p. 81-90, incorporate by reference. The methods disclosed therein can be used to prepare posttranslationally modified pertactin proteins that contain an attached lipid moiety.

Furthermore, in another embodiment, an immunogenic composition comprising *Bordetella bronchiseptica* and an isolated Bsp22 antigen. In another embodiment, the immunogenic composition comprises *Bordetella bronchiseptica,* an isolated pertactin antigen and further comprises an isolated Bsp22 antigen. The Bsp22 antigen can be prepared as provided in Medhekar et al., Molecular Microbiology (2009) 71(2), 492-504. Preferably, the isolated Bsp22 antigen is present in conjunction with (i.e. in addition to) a *Bordetella bronchiseptica* extract and an isolated pertactin antigen, specifically recombinant p68. "Bsp22" also includes lipidated forms of the antigen. Examples of production of lipidated proteins is provided in Erdile et al., Infection and Immunity, (1993) v.61(1) p. 81-90, incorporated by reference. The methods disclosed therein can be used to prepare posttranslationally modified Bsp22 proteins that contain an attached lipid moiety.

Viruses encompassed by the present invention can be propagated in cells, cell lines and host cells. Said cells, cell lines or host cells can be for example, but not limited to, mammalian cells and non-mammalian cells, including insect and plant cells. Cells, cell lines, and host cells in which viruses encompassed by the present invention can be propagated are readily known, and accessible to those of ordinary skill in the art.

Bacteria encompassed by the present invention can be cultured and propagated using various culture media known to those of ordinary skill in the art, including both broth (liquid) and agar (solid; semi-solid) cultivation media. Some bacteria can also be cultured and propagated in mammalian cells or non-mammalian cells.

The viruses and bacteria encompassed by the present invention can be attenuated or inactivated prior to use in an immunogenic composition or vaccine. Methods of attenuation and inactivation are well known to those skilled in the art. Methods for attenuation include, but are not limited to, serial passage in cell culture on a suitable cell line (viruses and some bacteria), serial passage in broth culture (bacteria), ultraviolet irradiation (viruses and bacteria), and chemical mutagenesis (viruses and bacteria). Methods for viral or bacterial inactivation include, but are not limited to, treatment with formalin, betapropriolactone (BPL) or binary ethyleneimine (BEI), or other methods known to those skilled in the art.

Inactivation by formalin can be performed by mixing the suspension containing the microorganism with 37% formaldehyde to a final formaldehyde concentration of 0.5%. The microorganism-formaldehyde mixture is mixed by constant stirring for approximately 24 hours at room temperature. The inactivated microorganism mixture is then tested for residual live organisms by assaying for growth on a suitable cell line or broth media.

For some antigens, inactivation by BEI can be performed by mixing the suspension containing the microorganism of the present invention with 0.1 M BEI (2-bromo-ethylamine in 0.175 N NaOH) to a final BEI concentration of 1 mM. For other antigens, the final BEI concentration is 2 mM. One skilled in the art would know the appropriate concentration to use. The virus-BEl mixture is mixed by constant stirring for approximately 48 hours at room temperature, followed by the addition of 1.0 M sodium thiosulfate to a final concentration of 0.1 mM. Mixing is continued for an additional two hours. The mixture containing the inactivated microorganism is tested for residual live virus by assaying for growth on a suitable cell line or broth media.

Immunogenic compositions and vaccines encompassed by the present invention can include one or more veterinarily-acceptable carriers. As used herein, a "veterinarily-acceptable carrier" includes any and all solvents, dispersion media, coatings, adjuvants, stabilizing agents, diluents, preservatives, antibacterial and antifungal agents, isotonic agents, adsorption delaying agents, and the like. Diluents can include water, saline, dextrose, ethanol, glycerol, and the like. Isotonic agents can include sodium chloride, dextrose, mannitol, sorbitol, and lactose, among others known to those skilled in the art. Stabilizers include albumin, among others known to the skilled artisan. Preservatives include merthiolate, among others known to the skilled artisan.

The adjuvant can be metabolizable, referring to adjuvants consisting of components that are capable of being metabolized by the target species such as vegetable oil based adjuvants. A metabolizable adjuvant can be a metabolizable oil. Metabolizable oils are fats and oils that typically occur in plants and animals, and usually consist largely of mixtures of triacylglycerols, also known as triglycerides or neutral fats. These nonpolar, water insoluble substances are fatty acid triesters of glycerol. Triacylglycerols differ according to the identity and placement of their three fatty acid residues or side chains.

The adjuvant can also be non-metabolizable, referring to adjuvants consisting of components that cannot be metabolized by the body of the animal subject to which the emulsion is administered. Non-metabolizable oils suitable for use in compositions of the present invention include alkanes, alkenes, alkynes, and their corresponding acids and alcohols, the ethers and esters thereof, and mixtures thereof. Preferably, the individual compounds of the oil are light hydrocarbon compounds, i.e., such components have 6 to 30 carbon atoms. The oil can be synthetically prepared or purified from petroleum products. Preferred non-metabolizable oils for use in compositions described herein include mineral oil, paraffin oil, and cycloparaffins, for example. The term "mineral oil" refers to a non-metabolizable adjuvant oil that is a mixture of liquid hydrocarbons obtained from petrolatum via a distillation technique. The term is synonymous with "liquefied paraffin", "liquid petrolatum" and "white mineral oil." The term is also intended to include "light mineral oil," i.e., oil which is similarly obtained by distillation of petrolatum, but which has a slightly lower specific gravity than white mineral oil. Mineral oil can be obtained from various commercial sources, for example, J.T. Baker (Phillipsburg, PA), USB Corporation (Cleveland, OH). Light mineral oil is commercially available under the name DRAKEOL®.

Adjuvants include, but are not limited to, the Emulsigen adjuvant system (MVP Laboratories; Ralston, NE), the RIBI adjuvant system (Ribi Inc.; Hamilton, MT), alum, aluminum hydroxide gel, oil-in water emulsions, water-in-oil emulsions such as, e.g., Freund's complete and incomplete adjuvants, Block copolymer (CytRx; Atlanta, GA), SAF-M (Chiron; Emeryville, CA), AMPHIGEN® adjuvant, saponin, Quil A, QS-21 (Cambridge Biotech Inc.; Cambridge, MA), GPI-0100 (Galenica Pharmaceuticals, Inc.; Birmingham, AL) or other saponin fractions, monophosphoryl lipid A, Avridine lipid-amine adjuvant, heat-labile enterotoxin from *E. coli* (recombinant or otherwise), cholera toxin, muramyl dipeptide, squalene/pluronic block copolymer/surfactant (SP-oil), sulpholipobeta-cyclodextrin (SL-CD), liposomes containing an immumodulator (e.g., CpG or poly I:C), muramyl dipeptide (MDP), iscomatrix (Quil A/phosphotidyl choline), CpG/DEAE-dextran/mineral oil (TXO), CpG, triterpenoids (e.g., Quil A or another purified or partially purified saponin preparation), sterols (e.g., cholesterol), immunomodulatory agents (e.g., dimethyl dioctadecyl ammonium bromide - DDA), polymers (e.g., polyacrylic acid such as CARBOPOL®), and Th2 stimulants (e.g., glycolipids such as Bay R1005®), and combinations thereof, among many other adjuvants known to those skilled in the art.

Non-limiting examples of various combinations that can be used include a triterpenoid plus a sterol (e.g., Quil A/cholesterol, also known as QAC), a triterpenoid plus a sterol, an immunomodulatory agent, and a polymer (e.g., Quil A/cholesterol/DDA/CARBOPOL®, also known as QCDC), and a triterpenoid plus a sterol, an immunomodulatory agent, a polymer, and a Th2 stimulant (e.g., Quil A/cholesterol/DDA/CARBOPOL®, and Bay R1005®, also known as QCDCR).

The amounts and concentrations of adjuvants and additives useful in the context of the present invention can readily be determined by the skilled artisan. In one embodiment, the present invention contemplates immunogenic compositions and vaccines comprising from about 20 µg to about 2000 µg of adjuvant. In another embodiment, adjuvant is included in an amount from about 100 µg to about 1500 µg, or from about 250 µg to about 1000 µg, or from about 350 µg to about 750 µg. In another embodiment, adjuvant is included in an amount of about 500 µg/2 ml dose of the immunogenic composition or vaccine.

The immunogenic compositions and vaccines can also include antibiotics. Such antibiotics include, but are not limited to, those from the classes of aminoglycosides, carbapenems, cephalosporins, glycopeptides, macrolides, penicillins, polypeptides, quinolones, sulfonamides, and tetracyclines. In one embodiment, the present invention contemplates immunogenic compositions and vaccines comprising from about 1 µg/ml to about 60 µg/ml of antibiotic. In another embodiment, the immunogenic compositions and vaccines comprise from about 5 µg/ml to about 55 µg/ml of antibiotic, or from about 10 µg/ml to about 50 µg/ml of antibiotic, or from about 15 µg/ml to about 45 µg/ml of antibiotic, or from about 20 µg/ml to about 40 µg/ml of antibiotic, or from about 25 µg/ml to about 35 µg/ml of antibiotic. In yet another embodiment, the immunogenic compositions and vaccines comprise less than about 30 µg/ml of antibiotic.

Immunogenic compositions and vaccines encompassed by the present invention can include one or more polynucleotide molecules encoding for a virus or bacteria, or viral or bacterial protein. DNA or RNA molecules can be used in immunogenic compositions or vaccines. The DNA or RNA molecule can be administered absent other agents, or it can be administered together with an agent facilitating cellular uptake (e.g., liposomes or cationic lipids). Total polynucleotide in the immunogenic composition or vaccine will generally be between about 0.1 µg/ml and about 5.0 mg/ml. In another embodiment, the total polynucleotide in the immunogenic composition or vaccine will be from about 1 µg/ml and about 4.0 mg/ml, or from about 10 µg/ml and about 3.0 mg/ml, or from about 100 µg/ml and about 2.0 mg/ml. Vaccines and vaccination procedures that utilize nucleic acids (DNA or mRNA) have been well described in the art, for example, U. S. Pat. No. 5,703,055, U.S. Pat. No. 5,580,859, U.S. Pat. No. 5,589,466, all of which are incorporated herein by reference.

In addition to the viruses or bacteria described above, immunogenic compositions and vaccines encompassed by the present invention can include other additional antigens. Antigens can be in the form of an inactivated whole or partial preparation of the microorganism, or in the form of antigenic molecules obtained by genetic engineering techniques or chemical synthesis. Other antigens appropriate for use in accordance with the present invention include, but are not limited to, those derived from pathogenic viruses such as canine distemper virus, canine herpesvirus, canine influenza virus, rabies virus, pathogenic bacteria such as *Leptospira bratislava, Leptospira canicola, Leptospira grippotyphosa, Leptospira icterohaemorrhagiae, Leptospira pomona, Leptospira hardjobovis, Porphyromonas* spp., *Bacteriodes* spp., *Borrelia* spp., *Streptococcus* spp., including *Streptococcus equi* subspecies *zooepidemicus, Ehrlichia* spp., *Mycoplasma* spp., including *Mycoplasma cynos,* and *Microsporum canis.* Antigens can also be derived from pathogenic fungi such as *Candida,* protozoa such as *Cryptosporidium parvum, Neospora caninum, Toxoplasma gondii, Eimeria* spp., *Babesia* spp., *Giardia* spp., *Leishmania* spp., or helminths such as *Taenia, Cuterebra, Echinococcus,* and *Paragonimus* spp.

### Forms, Dosages, Routes of Administration

Immunogenic compositions and vaccines encompassed by the present invention can be administered to animals to induce an effective immune response against CIRDC. Accordingly, the present invention provides methods of stimulating an effective immune response by administering to an animal a therapeutically effective amount of an immunogenic composition or vaccine described herein.

Immunogenic compositions and vaccines described herein can be administered to an animal to vaccinate the animal subject against CIRDC. The immunogenic compositions and vaccines can be administered to the animal to prevent or treat CIRDC in the animal. Accordingly, described herein are methods of vaccinating an animal against CIRDC, and preventing or treating CIRDC, comprising administering to the animal a therapeutically effective amount of an immunogenic composition or vaccine described herein.

Immunogenic compositions and vaccines encompassed by the present invention can be made in various forms depending upon the route of administration. For example, the immunogenic compositions and vaccines can be made in the form of sterile aqueous solutions or dispersions suitable for injectable use, or made in lyophilized forms using freeze-drying techniques. Lyophilized immunogenic compositions and vaccines are typically maintained at about 4°C, and can be reconstituted in a stabilizing solution, e.g., saline or HEPES, with or without adjuvant. Immunogenic compositions and vaccines can also be made in the form of suspensions or emulsions.

Immunogenic compositions and vaccines of the present invention include a therapeutically effective amount of one or more of the above-described microorganisms. Purified viruses and/or bacteria can be used directly in an immunogenic composition or vaccine, or can be further attenuated, or inactivated. Typically, an immunogenic composition or vaccine contains between about 1×10² and about 1×10¹² viral or bacterial particles, or between about 1×10³ and about 1×10¹¹ particles, or between about 1×10⁴ and about 1×10¹⁰ particles, or between about 1×10⁵ and about 1×10⁹ particles, or between about 1×10⁶ and about 1×10⁸ particles. The precise amount of a microorganism in an immunogenic composition or vaccine effective to provide a protective effect can be determined by a skilled artisan.

The immunogenic compositions and vaccines generally comprise a veterinarily-acceptable carrier, in a volume of between about 0.5 ml and about 5 ml. In another embodiment the volume of the carrier is between about 1 ml and about 4 ml, or between about 2 ml and about 3 ml. In another embodiment, the volume of the carrier is about 1 ml, or is about 2 ml, or is about 5 ml. Veterinarily-acceptable carriers suitable for use in immunogenic compositions and vaccines can be any of those described hereinabove.

Those skilled in the art can readily determine whether a virus or bacteria needs to be attenuated or inactivated before administration. In another embodiment of the present invention, a virus or bacterium can be administered directly to an animal without additional attenuation. The amount of a microorganism that is therapeutically effective can vary, depending on the particular microorganism used, the condition of the animal and/or the degree of infection, and can be determined by a skilled artisan.

In accordance with the methods of the present invention, a single dose can be administered to animals, or, alternatively, two or more inoculations can take place with intervals of from about two to about ten weeks. Boosting regimens can be required, and the dosage regimen can be adjusted to provide optimal immunization. Those skilled in the art can readily determine the optimal administration regimen.

Immunogenic compositions and vaccines can be administered directly into the bloodstream, into muscle, into an internal organ, or under the skin. Suitable means for parenteral administration include intravenous, intraarterial, intramuscular, and subcutaneous administration. Suitable devices for parenteral administration include needle (including microneedle) injectors and needle-free injectors.

Parenteral formulations are typically aqueous solutions which can contain excipients such as salts, carbohydrates, proteins, and buffering agents (preferably to a pH of from about 3 to about 9, or from about 4 to about 8, or from about 5 to about 7.5, or from about 6 to about 7.5, or about 7 to about 7.5), but, for some applications, they can be more suitably formulated as a sterile non-aqueous solution or as a dried form to be used in conjunction with a suitable vehicle such as sterile, pyrogen-free water or saline.

The preparation of parenteral formulations under sterile conditions, for example, by lyophilization, can readily be accomplished using standard pharmaceutical techniques well known to those skilled in the art.

The solubility of materials used in the preparation of parenteral solutions can be increased by the use of appropriate formulation techniques known to the skilled artisan, such as the incorporation of solubility-enhancing agents, including buffers, salts, surfactants, liposomes, cyclodextrins, and the like.

Compositions for parenteral administration can be formulated to be immediate or modified release. Modified release formulations include delayed, sustained, pulsed, controlled, targeted and programmed release. Thus, immunogenic compositions and vaccines can be formulated as a solid, semi-solid, or thixotropic liquid for administration as an implanted depot, providing modified release of the immunogenic compositions and vaccines.

Other means of immunogenic composition or vaccine administration include delivery by microneedle or needle-free (e.g. Powderject™, Bioject™, *etc.*) injection.

In cases where subcutaneous or intramuscular injection is used, an isotonic formulation is preferred. Generally, additives for isotonicity can include sodium chloride, dextrose, mannitol, sorbitol, and lactose. In particular cases, isotonic solutions such as phosphate buffered saline are used. The formulations can further encompass stabilizers such as gelatin and albumin. In some embodiments, a vaso-constrictive agent is added to the formulation. The pharmaceutical preparations according to the present invention are generally provided sterile and pyrogen-free. However, it is well known by those skilled in the art that the formulations for the pharmaceutically accepted carrier are those pharmaceutical carriers approved in the regulations promulgated by the United States Department of Agriculture, or equivalent government agency in a foreign country such as Canada or Mexico, or any one of the European nations, for any canine vaccine, polypeptide (antigen) subunit immunogenic compositions and vaccines, recombinant virus vector vaccines, and DNA vaccines. Therefore, the pharmaceutically accepted carrier for commercial production of the immunogenic compositions or vaccines is a carrier that is already approved or will be approved by the appropriate government agency in the United States of America or foreign country. The immunogenic compositions and vaccines can further be mixed with an adjuvant that is pharmaceutically acceptable. In certain formulations of the immunogenic compositions and vaccines, the immunogenic composition or vaccine is combined with other canine immunogenic compositions or vaccines to produce a polyvalent product that can protect canine against a wide variety of diseases caused by other canine pathogens.

### Detection and Diagnostic Methods

The extent and nature of the immune responses induced in the animal can be assessed by using a variety of techniques. For example, sera can be collected from the inoculated animals, and tested for the presence or absence of antibodies specific for the immunogens. Detection of responding cytotoxic T-lymphocytes (CTLs) in lymphoid tissues, indicative of the induction of a cellular immune response, can be achieved by assays such as T cell proliferation. The relevant techniques are well described in the art.

### Kits

Inasmuch as it may be desirable to administer an immunogenic composition or vaccine in combination with additional compositions or compounds- for example, for the purpose of treating a particular disease or condition- it is within the scope of the present invention that an immunogenic composition or vaccine can conveniently be included in, or combined in, the form of a kit suitable for administration or co-administration of the compositions.

Thus, kits encompassed by the present invention can comprise one or more separate pharmaceutical compositions, at least one of which is an immunogenic composition or vaccine in accordance with the present invention, and a means for separately retaining said compositions, such as a container, divided bottle, or divided foil packet. An example of such a kit is a syringe and needle, and the like. A kit of the present invention is particularly suitable for administering different dosage forms, for example, oral or parenteral, for administering the separate compositions at different dosage intervals, or for titrating the separate compositions against one another. To assist one administering a composition encompassed by the present invention, the kit typically comprises directions for administration.

Another kit encompassed by the present invention can comprise one or more reagents useful for the detection of an infected animal. The kit can include reagents for analyzing a sample for the presence of whole microorganisms, polypeptides, epitopes or polynucleotide sequences. The presence of virus, bacteria, polypeptides, or polynucleotide sequences can be determined using antibodies, PCR, hybridization, and other detection methods known to those of skill in the art.

Another kit encompassed by the present invention can provide reagents for the detection of antibodies against particular epitopes. Such reagents are useful for analyzing a sample for the presence of antibodies, and are readily known and available to one of ordinary skill in the art. The presence of antibodies can be determined using standard detection methods known to those of skill in the art.

In certain embodiments, the kits can include a set of printed instructions, or a label indicating that the kit is useful for the detection of infected animals.

### Antibodies

Antibodies can either be monoclonal, polyclonal, or recombinant. The antibodies can be prepared against the immunogen or a portion thereof. For example, a synthetic peptide based on the amino acid sequence of the immunogen, or prepared recombinantly by cloning techniques, or the natural gene product and/or portions thereof can be isolated and used as the immunogen. Immunogens can be used to produce antibodies by standard antibody production technology well known to those skilled in the art. Antibody fragments can also be prepared from the antibodies by methods known to those skilled in the art, and include Fab, F(ab')₂, and Fv fragments.

In the production of antibodies, screening for the desired antibody can be accomplished by standard methods in immunology known in the art. In general, ELISAs and Western blotting are the preferred types of immunoassays. Both assays are well known to those skilled in the art. Both polyclonal and monoclonal antibodies can be used in the assays. The antibody can be bound to a solid support substrate, conjugated with a detectable moiety, or be both bound and conjugated as is well known in the art. The binding of antibodies to a solid support substrate is also well known in the art. The detectable moieties contemplated for use in the present invention can include, but are not limited to, fluorescent, metallic, enzymatic and radioactive markers such as biotin, gold, ferritin, alkaline phosphatase, b-galactosidase, peroxidase, urease, fluorescein, rhodamine, tritium, ¹⁴C, and iodination.

The present invention is further illustrated by, but by no means limited to, the following examples.

### EXAMPLES

### Example 1: Immunogenicity of a Bordetella bronchiseptica bacterial extract, subunit vaccine.

Thirty-two approximately 8-week-old beagles with low *Bordetella bronchiseptica* (Micro-agglutinating antibody (MAT) titers of ≤16) were enrolled in the study. The puppies were divided randomly into two treatment groups (T1 and T2) of 16 each. One puppy from T2 was removed prior to first vaccination due to an inguinal hernia.

**Table 1. Study Design**

| Group | Vaccine | N | **Vaccination** | | | **Challenge** | | |
|---|---|---|---|---|---|---|---|---|
| | | | **Volume mL** | **Study Days** | **Route** | **Study Day** | **Dose/Dog** | **Route** |
| T1 | Saline | 16 | 1.0 | 0 and 21 | SC | 42 | 6.4x10⁸ CFU (colony forming unit) | Intranasal aerosolization in the chamber |
| T2 | *B. bronchiseptica* Extract-Subunit Vaccine | 15 | 1.0 | | | | | |

Dogs were vaccinated subcutaneously (SC) with the appropriate vaccine on Days 0 and 21, according to the study design shown in Table 1. The vaccines were administered to each dog in the right shoulder region for the first vaccination, and in the left shoulder region for the second vaccination.

On Day 42, dogs from all treatment groups were challenged intranasally with *B. bronchiseptica* using aerosolization in a Plexiglas chamber for 30 minutes. Four dogs, 2 from T1 and 2 from T2, were challenged at a time, with the exception of one group, which had only 3 animals, two from T1 and one from T2, because one animal was removed earlier from the study.

The primary efficacy variable was cough. All other variables (nasal discharge, ocular discharge, sneezing, depression, retching, respiration) were considered supporting secondary variables. Clinical observations for signs of respiratory disease, including cough, were performed twice daily ("observation periods"; a.m. and p.m.), for approximately 30 minutes in each room per each session, from Day 42 and until Day 70 (conclusion of the study). Whether or not an animal coughed for two consecutive observation periods post-challenge was calculated for each animal. A frequency distribution of coughing/not coughing for two consecutive observation periods was calculated for each treatment. Coughing/not coughing for two consecutive observation periods was analyzed with a Cohran-Armitage test, adjusting for room since it was not possible to analyze the data with a generalized linear mixed model. Frequency distributions of nasal bacterial isolation (+/-) were calculated for each treatment and time point. It was also determined for each animal whether or not it ever had bacteria isolated post-challenge.

A frequency distribution of whether an animal ever had *B. bronchiseptica* isolated post challenge was calculated for each treatment, and was analyzed with a generalized linear mixed model, if possible. Duration of nasal shedding post-challenge was calculated for each animal, and was analyzed with a general linear mixed model. Antibody titers were logarithmically transformed, and analyzed using a general linear mixed model for repeated measures.

Results: There was no pain or fever (≥39.5°C) reported in any of the dogs following vaccination. Some dogs in both treatment groups were reported to have scratching at the time of vaccination. Small size injection swellings (2x2x1 cm) were reported in only two dogs in T2 during the 3-6 hours post first vaccination observation. The data indicate that the vaccine was safe and well-tolerated by the dogs.

All saline controls developed cough, indicating adequate challenge. The least square (LS) means for the duration of cough in the control group was 20.3. By contrast, the vaccine significantly reduced the duration of cough to LS means of 5.2 in the vaccinates with p-value <.0001. Additionally, other respiratory clinical signs, such as retching and respiration, were reduced in the vaccinates when compared to the controls.

Post-challenge bacterial isolation data showed that the test vaccine significantly reduced the duration of nasal shedding of the challenge organism in the vaccinate group (LS mean of 20.5) when compared to the saline controls (LS mean of 25.3) with a p-value 0.0061. The data indicate that the vaccine is effective in reducing the level of infection, as confirmed by the reduced duration of shedding in the vaccinates.

The antibody responses generated post vaccination (Days 21 and 42) in the T2 group were similar to T1, likely due to the nature of the agglutinating antibody assay, and not lack of antigenicity. A robust anamnestic response was induced in the majority of vaccinates post-challenge at day 70 was observed (LS mean 426) compared to saline control (LS mean 13), indicating effective immunization.

Taken together, the data from this study clearly indicate that the *Bordetella bronchiseptica* bacterial extract, subunit vaccine demonstrated efficacy, by the reduced duration of cough and nasal shedding.

### Example 2: Evaluation of the efficacy and safety of an injectable Bordetella bronchiseptica vaccine.

Fifty beagle dogs, at approximately 8 weeks of age, were enrolled in the study. The dogs were divided into 5 groups of 10 each. All animals were in good general health, and did not receive any *Bordetella* vaccinations. Prescreen serum samples were negative for *B. bronchiseptica,* with titers <16 by the Micro Agglutination Test (MAT) prior to first vaccination. All animals were determined free of *B. bronchiseptica* by a bacterial nasal swab isolation test prior to first vaccination (Day 0).

**Table 2. Study Design**

| Group | Investigational Veterinary Product (IVP) | N | **Vaccination** | | | **Challenge** | | |
|---|---|---|---|---|---|---|---|---|
| | | | **Volum e mL** | **Study Days** | **Rout e** | **Stud y Day** | **Dose/D og** | **Route** |
| T01 | Saline | 1 0 | 1.0 | 0 and 21 | SC | 42 | 5.4x10⁸ CFU (colony forming unit) | Intranasal aerosolizatio n in the chamber |
| T02 | *B. bronchiseptica* bacterial extract / Pertactin (10µg)/QCDC | 1 0 | 1.0 | 0 and 21 | SC | | | |
| T03 | Modified Commercial adjuvanted *B. bronchiseptica* extract vaccine | 1 0 | 1.0 | 0 and 21 | SC | | | |
| T04 | Pertactin (10µg) /QCDC | 1 0 | 1.0 | 0 and 21 | SC | | | |
| T05 | Commercial intranasal vaccine | 1 0 | 0.5 | 0 | IN | | | |

Animals were vaccinated with the appropriate vaccine on Days 0 and 21, according to the study design shown in Table 2. The vaccines for groups T01, T02, T03, and T04 were administered subcutaneously to each dog in the right shoulder region for the first vaccination, and in the left shoulder region for the second vaccination. Group T05 received a single intranasal vaccination of a commercially-available intranasal vaccine on Day 0. This group was vaccinated last to prevent spread of live vaccine in the premises, and dogs in this group were housed separately in a different room to prevent exposure to other groups.

On Day 42, dogs from all treatment groups were challenged intranasally with *Bordetella bronchiseptica* by aerosolization in a Plexiglas chamber for a total of 30 minutes. Five dogs from the same pen (one from each treatment group) were challenged at a time.

The primary efficacy variable was cough; bacterial isolation was the secondary variable. Clinical observations for signs of respiratory disease, including cough, were performed twice daily ("observation periods"; a.m. and p.m.), for approximately 30 minutes in each room per each session, from Day 42 and until Day 62 and once (a.m.) on Day 63 (conclusion of the study). Prior to cough analysis, the percentage of observation periods coughed were transformed with an arcsine square root transformation. The transformed percentage of observation periods, and number of days with cough, were analyzed with a general linear mixed model. The percentage of observation periods post-challenge in which an animal coughs, and the number of days post-challenge with cough, were calculated for each animal.

Results: Mild to moderate swellings were observed at the injection sites in dogs of treatment group T02 and T04 (data not shown). The severity of the reactions and the number of dogs involved increased after the second vaccination. Mild injection site reactions were observed in a few dogs in treatment group T03. No measurable injections swelling were observed in the saline group T01. There was no clinical fever (≥ 39.5°C) observed, except for one dog in Group T02 on Day 4 post-vaccination. There was no pain reported in any of the vaccinated dogs.

The *B. bronchiseptica* challenge inoculation induced cough in all unvaccinated controls (53.3% and 14.7 days coughed), indicating the challenge was adequate to evaluate the test vaccines. The results obtained for Group T05, which received the commercial intranasal vaccine (positive control), displayed 4.6% observed cough up to 2 days post challenge, suggesting that the level of challenge is optimum and not overwhelming.

Three test formulations were evaluated in this study. T02 had 16.1% observed cough up to 6 days post challenge, T03 had 34.7% observed cough up to 10.5 days post challenge and T04 had 34.5% observed cough up to 10.7 days post challenge. Accordingly, all vaccinated groups showed reduced cough scores when compared to the unvaccinated controls. Group T02 vaccinated with a *B. bronchiseptica* bacterial extract, spiked with pertactin and adjuvanted with QCDC, demonstrated a statistically significant reduction in the cough score criteria. Thus, the efficacy of the formulation tested in T02 appeared efficacious.

### Example 3. Safety and Efficacy of Bordetella bronchiseptica-Containing Vaccines in Dogs.

Fifty (50) dogs, divided into 5 treatment groups, were selected for the study. Animals were determined to be fit for the study based on a physical examination on Day -4,

Blood samples (approximately 8 mL) for serology were collected in SST tubes from all animals on Study Days -2, 21 and 28 prior to each vaccination. The serum samples collected on Day -2 were used to confirm animals were free of B. *bronchiseptica.* Nasal swabs were collected prior to vaccination on Day 0, and tested for the presence of *B. bronchiseptica.* Tympanic temperatures were collected starting on Day -4, to establish a baseline prior to vaccination.

Animals were vaccinated with the appropriate vaccine on Days 0, 21, and 28 according to the study design shown in Table 3. The vaccines were administered subcutaneously to each dog in the right shoulder region for the first vaccination, and in the left shoulder region for the second vaccination.

**Table 3. Study Design**

| **Group** | **IVP** | **N** | **Vaccination¹** | | | **Challenge²** | | |
|---|---|---|---|---|---|---|---|---|
| | | | **Vol (mL)** | **Study Days** | **Route** | **Study Day** | **Target Dose/Dog** | **Route** |
| T01 | *B. bronschiseptica* (inactivated) + Pertactin (10µg) No Adjuvant | 10 | 1.0 | 0 and 28 | SC | 56 | 10⁹ | Intranasal (aerosol; chamber) |
| T02 | Saline | 10 | 1.0 | 0 and 28 | | | | |
| T03 | *B. bronschiseptica* (inactivated) + Pertactin (10µg) No Adjuvant | 10 | 1.0 | 0 and 21 | | | | |
| T04 | CRCoV/CIV/CPIV/ CAV2 rehydrated with ***B***. *bronschiseptica* (inactivated) + Pertactin (10µg) No Adjuvant | 10 | 1.0 | 0 and 28 | | | | |
| T05 | CRCoV/CIV/CPIV/ CAV2 rehydrated with water (diluent) | 10 | 1.0 | 0 and 28 | | | | |

| | | | | | | | | |
|---|---|---|---|---|---|---|---|---|
| ¹ Investigational Veterinary Product (IVP) was administered (SC) subcutaneously. ² Target challenge dose of 10^9 organisms of *Bordetella bronchiseptica* strain. | | | | | | | | |

All animals were observed on vaccination Days 0, 21, and 28 for injection site reactions following vaccination. They were observed daily for injection reactions post vaccination from Days 1 to 7 and 22-35. Tympanic temperatures were collected on Days 0 to 7 and 21 to 35.

Blood samples (approximately 6 mL) for serology were collected on Day 55, one day prior to challenge. Tympanic temperatures were collected on Days 54, 55, and 56 prior to challenge. Nasal swabs were collected on Day 55, one day prior to challenge, and tested for the presence of *B. bronchiseptica.* Animals were observed twice daily (a.m. and p.m.), approximately 30 minutes each session on Days 54 and 55, and in the a.m. on Day 56, for clinical signs of respiratory disease, in order to establish baseline values.

*Bordetella bronchiseptica* challenge strain) was used to prepare a target challenge dose of 10⁹ CFU/4 mL/dog. On Day 56, dogs from all treatment groups were challenged intranasally with B. *bronchiseptica* by aerosolization in a Plexiglas chamber for a total of 30 minutes for each pen challenged. Five dogs from the same pen (one from each treatment group) were challenged at a time.

Tympanic temperatures was recorded once daily after challenge from Days 56 to 77. Clinical observations were performed twice daily (a.m. and p.m.), for approximately 30 minutes in each room per each session, from Day 56 and until Day 76 and once (a.m.) on Day 77. Briefly, cough, nasal discharge, sneeze, ocular discharge, retch, and depression were observed using the following scoring system: Absent (0), Mild (1), Moderate (2), and Severe (3). Nasal swabs were collected on Days 59, 62, 66, 69, 74, 76 and 77, to determine shedding of challenge organisms.

Blood samples (approximately 6 mL) for serology were collected on Day 77. Nasal swabs for isolation of *B. bronchiseptica* were collected using swabs and transport media.

Agglutinating antibodies to *B. bronchiseptica* were determined by the Micro Agglutination Test (MAT). Serum samples from treatment groups T04 and T05 from Days 0, 28, 55, and 77 were titrated for CRCoV antibodies by serum neutralization and IFA, and for CIV by HAI. *B. bronchiseptica* isolation from nasal swabs was performed according to standard procedure. Each sample was tested qualitatively for the presence or absence of bacteria.

Results. Fifty (50) healthy approximately 8-week-old beagle puppies were confirmed by nasal swab culture isolation to be free of *B. bronchiseptica* organisms on Day 0. Serum samples evaluated for *B. bronchiseptica* agglutinating antibodies by the MAT confirmed that all puppies were susceptible with MAT titers of ≤8 on Day -2.

All experimental vaccines evaluated in this study produced mild to no injection swellings after the first vaccination. Injection swellings were limited to study day 0 for the majority of vaccinates. Mild to no injection swellings were also reported after the second vaccination. The injection site swellings when they occurred, resolved between one to three days after the second vaccination. Scratching was reported predominantly in the 5-way combination group (T04). There was no clinical fever reported after vaccinations. There were no injection swellings reported in the saline group. The data confirmed the safety of the vaccines.

The colony count performed before and after challenge inoculation confirmed that an average of 1.45 x 10⁸ CFU *Bordetella* per dog were aerosolized in the chamber. Challenge inoculation induced cough in all saline control dogs (T02) with a mean percentage observation coughed of 43.5% and 12.2 days coughed. Treatment group T05, vaccinated with 4-way viral only (CRCoV/CIV/CPIV/CAV2) without *Bordetella* antigen developed cough similar to the saline control with a mean percentage observation coughed of 43.4% and 12.2 days coughed. These findings indicate that the challenge was adequate and consistent to evaluate the test vaccines.

Dogs in treatment group T01 vaccinated with the *Bordetella* vaccine were significantly protected against challenge (3.6 days coughed, p<0.0001) when compared to the control group (12.2 days coughed). The same vaccine also significantly protected dogs in T03 when given at 3-weeks interval regimen (5.8 days coughed, p=0.0004). The reduction in cough scores in these two groups (T01 vs T03) was not significantly different (p-value=0.1883) suggesting that the level of protection for the vaccine given with a 3 or 4 weeks interval, is similar.

Dogs in T04 that received the non-adjuvanted 5-way combination vaccine were significantly (p=0.0016) protected against *Bordetella* challenge (6.6 days coughed) when compared to the saline controls (12.2 days coughed), and when compared to T05 receiving the 4-way viral (CRCoV/CIV/CPIV/CAV2) combination (12.1 days coughed, p=0.0019) indicating efficacy of the *Bordetella* fraction in the combination vaccine lacking adjuvant.

Serological evaluation of the viral fractions in the 5-way combination vaccine was possible for only two fractions, the CIV and CRCoV, where dogs were confirmed seronegative on study day -2. CIV HAI response in the 4-way vaccine group (T04) on study day 56 were numerically similar to that in the 5-way vaccine group (T05) and indicate lack of interference by the *Bordetella* fraction on the CIV antigen. CRCoV SN responses on study day 56 were numerically higher in the 4-way vaccine group (T04) than in the 5-way vaccine group (T05), indicating possible interference by the *Bordetella* on the CRCoV fraction. However, these findings are not conclusive since these vaccines were not adjuvanted and the formulation was not optimized and CRCoV challenge was not conducted to test efficacy.

The monovalent *Bordetella* vaccine was confirmed to be safe and efficacious. The efficacy of the monovalent vaccine was demonstrated when the vaccine was given at 21 or 28 days intervals. The *Bordetella* fraction was also shown to be efficacious when give in a 5-way non-adjuvanted combination vaccine.

### Example 4. Multivalent serology study

Forty dogs, approximately 8 weeks of age and in good general health, were pre-screened for *Bordetella bronchiseptica* by Micro Agglutination Test (MAT), and for canine respiratory coronavirus (CRCoV) by indirect fluorescent antibody assay (IFA). Serum neutralization (SN) was also used to evaluate antibody levels. On Day 0, all dogs were negative for antibodies to *Bordetella bronchiseptica* as determined by MAT (≤16), and negative for antibodies to CRCoV as determined by IFA (<40). All dogs were also free of *Bordetella bronchiseptica* and CRCoV, as determined by nasal swab isolation test prior to first vaccination (Day 0).

Dogs were divided into 5 treatment groups of 8 dogs each, and vaccinated according to the study design shown in Table 4. The vaccines were administered to each dog in the right shoulder region for the first vaccination, and in the left shoulder region for the second vaccination.

**Table 4. Study Design**

| **Treatment Group** | **Investigational Veterinary Product (IVP)** | **Adjuvant** | **N** | **Vaccination¹** | |
|---|---|---|---|---|---|
| | | | | **Study Days** | **Route** |
| T01 | CAV2/CPIV/CPV/L4 | 5% Rehydragel | 8 | 0 and 21 | Subcutaneously (SC) |
| T02 | CAV-2, CPI, CRCoV+ *Bordetella,* CIV | QCDC | 8 | | |
| T03 | CAV-2, CPI, CRCoV+ *Bordetella,* CIV | 1% EMA¹/ 3% Neocryl/ 5% Emulsigen SA | 8 | | |
| T04 | CAV-2, CPI, CRCoV+ *Bordetella,* CIV | QCDC | 8 | | |
| T05 | CAV-2, CPI, CRCoV+ *Bordetella,* CIV | QCDC | 8 | | |

| | | | | | |
|---|---|---|---|---|---|
| ¹ EMA= ethylene maleic anhydride | | | | | |

Following the second vaccination, due to complications, groups T04 and T05 were removed from the study. Dogs in the remaining groups (T01, T02, and T03) were observed daily for post vaccination reactions, and monitored for body (tympanic) temperature for 7 days after each vaccination. Blood samples were collected from dogs on Days 0, 21, 42 and 56 to measure antibody responses.

Serum samples from Day 0, 21, 42 and 56 were tested for agglutinating antibodies to *Bordetella bronchiseptica* by the MAT assay. Serum samples from the same days were also titrated for CRCoV antibodies by serum neutralization, for CIV by HAI, and for CAV-2 and CPI antibodies by serum neutralization. Geometic mean antibody titers were obtained for each treatment group.

### Results:

The test vaccines in groups T02 and T03 induced antibody responses in all (100%) the vaccinated dogs after the second dose, indicating active immunization against the viral antigens. The antibody response increased after the second vaccination in the majority of vaccinated dogs, indicating a booster effect of the second vaccination. It is important to note that the antibody responses among the viral fractions was achieved in the presence of multiple viral and bacterial (*B. bronchiseptica*) antigens, indicating lack of immunological interference. The MAT serology is not correlative to protection against *Bordetella,* but is rather a valuable screening tool to enroll suitable study animals. In conclusion, based on the immunological response in vaccinated dogs, efficacy of the viral antigens is predicted in the 5 way multivalent vaccine.

### Example 5. Duration of immunity study

### A. Monovalent:

The purpose of this study is to demonstrate the duration of immunity of a *Bordetella bronchiseptica* extract-subunit vaccine in 8-week-old dogs against a virulent *B. bronchiseptica* challenge.

All animals are in good general health, and have not received any *Bordetella* vaccinations. Animals have low (≤16) or no antibody titers to *B. bronchiseptica* as determined by the Micro Agglutination Test (MAT) prior to first vaccination. All animals are also free of *B. bronchiseptica,* as determined by bacterial nasal swab isolation test prior to first vaccination.

Dogs are divided into 2 treatment groups; one group receives a placebo vaccine, and the other a *B. bronchiseptica* extract supplemented with a recombinant antigen. The antigen is pertactin, Bsp22, or both. Animals are vaccinated twice, approximately 3-4 weeks apart. They are observed for injection site reactions following each vaccination.

Approximately 6-12 months following vaccination, dogs from all treatment groups are challenged by aerosolization with *Bordetella bronchiseptica.* The challenge dose and purity of the challenge inoculum are determined before and after challenge. Clinical observations are performed leading up to and following challenge.

Nasal swabs for isolation of *B. bronchiseptica* are collected. Blood from each animal is collected for serum. Agglutinating antibodies to B. bronchiseptica are determined by the MAT. Serum samples are titrated for pertactin-specific IgG antibody response using ELISA. *B. bronchiseptica* isolation from nasal swabs is performed according to standard procedure.

Cough, *Bordetella* isolation (post-challenge), and post-vaccination serological response are criteria used to judge vaccine efficacy in the study.

### B. Multivalent:

The purpose of this study is to demonstrate the duration of immunity of a multivalent respiratory combination vaccine in dogs. The vaccine contains the following antigenic components: modified-live CAV-2, modified-live CPIV, inactivated CIV, inactivated CRCoV and a *Bordetella bronchiseptica* extract supplemented with a recombinant antigen, either pertactin, Bsp22, or both.

All animals are in good general health, and have not received any vaccinations for any of the pathogens for which the vaccine is designed to protect against. Dogs are divided into multiple sets of treatment groups. Each set consists of two treatment groups, a control group receiving a placebo vaccine, and a vaccinate group receiving the test vaccine. Animals are vaccinated twice, approximately 2-4 weeks apart. They are observed for injection site reactions following each vaccination.

Approximately 6-12 months following vaccination, each set of two treatment groups (vaccinates and controls) are challenged with one of the pathogens for which the vaccine is designed to protect against. Clinical observations are performed leading up to and following challenge. Nasal swabs for isolation of the challenge pathogen are collected during the post challenge period. Blood from each animal is collected for obtaining serum, which is used for subsequent analytical analysis. Clinical signs of respiratory disease, pathogen shedding post challenge, and serological responses are used as criteria to judge the efficacy of vaccines.

Having thus described in detail various embodiments of the present invention, it is to be understood that the invention defined by the appended claims is not to be limited to particular details set forth in the above description as many apparent variations thereof are possible without departing from the spirit or scope of the present invention.

All of the foregoing references are hereby incorporated by reference as if set forth fully herein.

## Claims

1. An immunogenic composition for use in the treatment or prevention of infection from a canine respiratory pathogen in a dog, the composition comprising *Bordetella bronchiseptica* cellular bacterial extract and an added isolated_*Bordetella bronchiseptica* p68 protein, wherein said p68 is present at between about 1 µg and about 30 µg.

2. The immunogenic composition for the use of claim 1, wherein said p68 is present at about 5 µg to about 20 µg.

3. The immunogenic composition for the use of claim 1, wherein said p68 protein is a recombinant p68 protein.

4. The immunogenic composition for the use of any one of claims 1 to 3, wherein said p68 is present at about 7 µg to about 15 µg.

5. The immunogenic composition for the use of any one of the previous claims, wherein said composition is non-adjuvanted.

6. The immunogenic composition for the use of any one claims 1-3, 5, wherein said p68 is present at about 20 µg.

7. The immunogenic composition for the use of any one of the previous claims, wherein said composition prevents infection from said canine respiratory pathogen in said dog for at least six months.

8. The immunogenic composition for the use of any one of the previous claims, wherein said composition prevents infection from said canine respiratory pathogen in said dog for about one year.

9. The immunogenic composition for the use of any one of the previous claims, wherein said composition further comprises a diluent or excipient for parenteral administration to a dog.

10. The immunogenic composition for the use of any one of the previous claims, wherein said composition further comprises an isolated Bsp22 antigen, optionally lipidated.

11. The immunogenic composition for the use of any one of the previous claims, wherein said composition further comprises an antigen from a canine respiratory pathogen selected from the group consisting of: canine parainfluenza virus (CPIV), canine adenovirus-2 (CAV-2), canine respiratory coronavirus (CRCoV), *Mycoplasma cynos* (*M. Cynos*) and canine influenza virus (CIV).

12. The immunogenic composition for the use of claim 11, wherein said composition comprises at least two, three or four of the antigens from the canine respiratory pathogen.

13. The immunogenic composition for the use of claim 11, wherein said composition comprises antigens from canine parainfluenza virus (CPIV), canine adenovirus-2 (CAV-2), canine respiratory coronavirus (CRCoV) and canine influenza virus (CIV).

14. The immunogenic composition for the use according to any one of claims 1 to 13 wherein the canine respiratory pathogen further comprises at least one, two, three or four of: canine parainfluenza virus (CPIV), canine adenovirus-2 (CAV-2), canine respiratory coronavirus (CRCoV) and canine influenza virus (CIV).

15. The immunogenic composition for the use according to any of claims 1 to 14 wherein the canine respiratory pathogen is canine infectious respiratory disease complex (CIRDC), wherein the composition treats or prevents infection from a plurality of said pathogens.
